# EUROPEAN PATENT SPECIFICATION

(11) **EP 1 637 169 B1**
(45) Date of publication and mention of the grant of the patent: **08.08.2007**
(21) Application number: 03727527.8
(22) Date of filing: 29.05.2003
(51) Int. Cl.: A61L 9/03, F23D 21/00

(54) **APPARATUS FOR DIFFUSION OF ACTIVE SUBSTANCES WITH CATALYTIC COMBUSTION**
GERÄT ZUR DIFFUSION VON WIRKSTOFFEN MIT KATALYTISCHER VERBRENNUNG
APPAREIL DE DIFFUSION DE SUBSTANCES ACTIVES A COMBUSTION CATALYTIQUE

(43) Date of publication of application: 22.03.2006
(73) Proprietor: Zobele Espana, S.A., 08290 Cerdanyola del Vallés (ES)
(72) Inventor: CASERTA, Andrea, 08290 Cerdanyola del Valles (ES); GARCIA FABREGA, Ruben, 08290 Cerdanyola del Valles (ES); MORHAIN, Cedric, 08290 Cerdanyola del Valles (ES)
(74) Representative: Carpintero Lopez, Francisco
(86) International application number: PCT/ES2003/000258
(87) International publication number: WO 2004/105812

(56) References cited:
- FR-A1- 2 179 664
- FR-A1- 2 483 782
- FR-A1- 2 680 118
- GB-A- 1 451 411
- US-A- 3 131 828
- US-A1- 2001 043 469

## Description

### OBJECT OF THE INVENTION

The present invention relates to an apparatus for diffusion of active substances with catalytic combustion comprising a container holding the fuel and active substances, which contains a wick connected to a burner that facilitates the catalytic combustion and the evaporation of the active substances, such as air fresheners, disinfectants, bactericides and others.

It is the object of the invention to provide the means to optimise the union between the burner and the wick, increase the safety of the device against possible spilling of the mixture, facilitate replenishing the mixture through an opening independent of that housing the burner, and incorporating means for indicating high temperatures.

### BACKGROUND OF THE INVENTION

Hitherto known are apparatuses for diffusion of active substances by the heat of a catalytic combustion comprising a container that holds a liquid fuel with active substances in solution, in which a wick is provided that leads the fuel to the combustion and evaporation area. The combustion area consists of a porous piece with a catalytic coating, while the evaporation area may be a part of this piece with a catalytic coating or an independent ceramic piece, or even a piece of a porous non-ceramic material.

The presence of the catalyst allows activating a chemical reaction of combustion of the mixture formed by fuel and active substances without the presence of a flame, which generates heat that causes the evaporation of the mixture in the evaporation area.

In the case of an application for Invention belonging to the holder of this invention, a device is disclosed for evaporation of active substances by catalytic combustion that comprises a fuel container and an active substance container, with independent wicks, one of the wicks having a burner that produces the heating and evaporation of the active substance, favoured by a heat diffuser.

In addition, it must be remarked that any molecule present in the air that comes in contact with the burner will be degraded and destroyed by the high temperature of the burner incorporated in one of these devices, so that molecules causing bad odours, tobacco smoke compounds, will be eliminated from the atmosphere. In some embodiments this effect is enhanced by placing a fan near the burner.

The document FR-A-2483782 describes an apparatus for diffusion of an active substance with catalytic combustion, comprising a catalytic burner having an opening for insertion of a wick elements.

A list of the main drawbacks presented by currently known apparatuses is given below:
- The container holding the fuel and active substances has a single opening that during normal operation constitutes the housing for the burner or ceramic piece in contact with the wick, while when the container is emptied constitutes the passage for refilling the mixture. This refilling operation requires the user to touch the burner and the wick, which are soaked in the mixture, with the ensuing risk of stains and direct contact with chemical products that should not come in contact with the skin.
- These apparatuses have low safety regarding the accidental spillage of the mixture of fuel and active substances, both while the apparatus is being used and between uses. In hitherto known apparatuses, the ceramic piece used as a support for combustion and evaporation is simply resting on the neck of the container without any mechanical attachment means. If the apparatus falls accidentally, the ceramic coupled to the wick can easily be displaced from its location and the mixture contained will be spilled. If this happens while the apparatus is operating with the ceramic piece in catalytic combustion at a high temperature, there is a risk of burns and/or fire.
- When the fuel in the apparatus is exhausted, the upper end of the wick is surrounded by the warm walls of the ceramic piece. The wick tends to char in the absence of oxygen, producing free carbon particles that obstruct the pores of the ceramic piece. To solve this problem, Invention Patent WO 9963267 includes in the ceramic piece a number of conducts communicating the wick to the external air.
- Apparatuses for diffusion of active substances by catalytic combustion include two caps, a protective cap to be used during operation and another cap to extinguish the burner by preventing access of oxygen to the combustion area. The extinguishing cap can be lost, as it cannot be attached to the apparatus while in use.
- While the apparatus is operating, the burner temperature is above 300°C without the presence of a flame nor any visible sign of its high temperature. Although apparatuses of this type are generally provided with a protector for the burner while in operation, the risk of accidental burns is considerable.

### DESCRIPTION OF THE INVENTION

The apparatus for diffusion of active substances with catalytic combustion object of this invention solves the drawbacks described above to full satisfaction on the basis of the characteristics indicated below.

The apparatus incorporates a container with two openings, a first opening meant to allow the mixture to pass and a second opening meant to attach the burner to the container, providing a clean use as it is not necessary to touch the burner or catalytic ceramic piece, nor to take out the soaked wick to refill the apparatus, as well as a safe use as the mixture will not spill if the apparatus falls accidentally during or after its use. This is provided by a permanent attachment of the burner and a refilling opening that can be closed well by a reusable cap.

In this apparatus the ceramic piece enters the upper end of the wick, not the other way round as is usual in other diffusers. This allows reducing the dimensions of the ceramic piece with two consequences: lowering the cost of the ceramic piece and reducing the consumption of the fuel - active substance mixture. An optimised designed of the ceramic piece determines an appropriate distribution of the catalytic and evaporation area, in order to provide an efficient diffusion of active substance, considerably reducing consumption and thereby reducing the number of refilling operations and optimising the performance of the product.

The ceramic piece is designed to be fixed to a metallic support that ensures its attachment to the container, while covering the second opening in which a centring element is housed that ensures the vertical position of the wick and a thermochromic element that indicates the proximity to the burner when it is operating at a high temperature. This metallic support ensures interference with the wick and protects the components inside it while the flame is present when the apparatus is turned on.

In addition, the support has a surface that can be, for example, 4 times greater than the exposed surface of the burner, dissipating effectively the heat generated by the burner so that its surface temperature is established without gradients at values under 100°C. This means that it is not necessary to manufacture the container out of refractory material, such as ceramic or glass, enabling to manufacture it out of a polymer to reduce its cost and further automate its manufacture, resulting in a piece with much smaller dimensional tolerances and thus a greater reliability of operation.

On another hand, as the temperature of this support is lower than 100°C, it is a suitable support for thermochromic pigments such as adhesives, paints or varnishes used to fix it.

The apparatus incorporates a cap for extinguishing the catalytic combustion that is placed inside the device; more specifically, it is placed on a refilling cap that covers the first opening, in order to ensure its availability and reduce the risk of misplacing it. When the burner must be extinguished, the extinguishing cap is moved to the burner, covering it.

Also foreseen is a protection incorporated in the diffuser apparatus that establishes a continuity of the container, so that it prevents a direct access to the burner when it is in operation, provided with orifices to allow the diffusion of the active substances evaporated.

Considered as an option is the incorporation of a regulation and/or interruption of the diffusion of the active substance by a relative displacement of the burner with respect to the wick, so that the amount of mixture arriving at the burner from the wick is changed or eliminated.

### DESCRIPTION OF THE DRAWINGS

To complete the description being made and in order to aid a better understanding of the characteristics of the invention, according to an example of a preferred embodiment, a set of drawings is accompanied as an integral part of the description where for purposes of illustration and in a non limiting sense the following is represented:

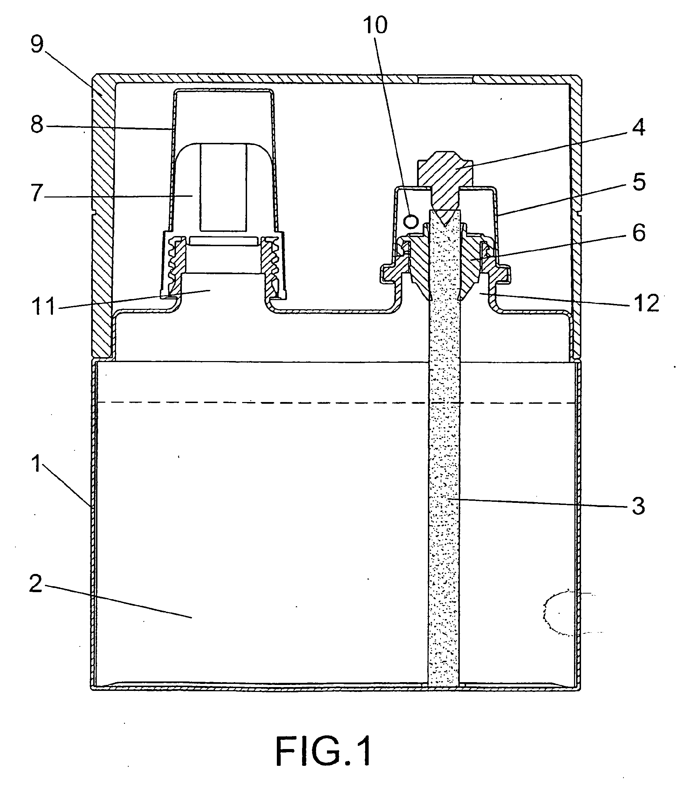

Figure 1.- Shows an elevation sectional view representing the elements constituting the apparatus for diffusion of active substances with catalytic combustion.

### PREFERRED EMBODIMENT OF THE INVENTION

The apparatus for diffusion of active substances with catalytic combustion that constitutes the object of this invention is based on the incorporation of a container (1) that holds a mixture (2) of active substance and fuel in which a wick (3) is placed that leads the mixture to a ceramic piece or burner (4) provided with a catalyst on its surface.

With this basic construction, the device mainly stands out-in that the container (1) incorporates two openings, a first opening (11) meant for refilling the mixture (2) in the container and a second opening (12) meant to allow the wick (3) to pass and to attach the burner (4), the burner (4) designed so that it has a conical-shaped lower end to allow coupling by insertion in the upper end of the wick (3) and so that it is coupled on a metallic support (5) that covers the second opening (12) in which is coupled a centring element (6) for the wick (3), which ensures the vertical position of said wick (3) in the correct position for interfering with the burner (4), being provided at the inlet opening (11) with a refilling cap (7) that closes the container after it is refilled.

Also noteworthy is the fact that the container (1) is covered by an external protection (9) that prevents contact with the burner (4) during its use, and is provided with orifices that allow circulation of air for combustion and evaporation of the active substances.

In addition, when the apparatus is being used a metallic extinguishing cap (8) is placed above the refilling cap (7), thereby ensuring its availability for a later use placing it on the metallic support (5) and covering the burner (4) to turn off the apparatus.

On the surface of the metallic support (5) of the ceramic piece it incorporates a thermochromic element (10) that changes colour to indicate an increase in the temperature of the metallic support (5) when the apparatus is in use, indicating this situation by either a colour code or an inscription representing heat.

The operation of the apparatus takes place according to the following sequence:

When the container is uncovered, with the mixture (2) of fuel and active substances inside it, the burner (4) is turned on with a flame. The flame is then extinguished, the catalytic combustion continuing without a flame so that the surface temperature of the burner or ceramic piece (4) reaches between 300 and 550°C; the external protector (9) is then set in place.

At the surface of the burner (4) with a catalytic coating the combustion is activated by the flame, the area without a coating acting only as a support for the evaporation of the mixture by the heat produced by the catalytic combustion, so that the active substance vapour exits through the orifices of the external protector (9).

To turn off the apparatus, the catalytic combustion process must be extinguished in one of two ways: preventing the access of oxygen by covering the burner (4) with the extinguishing cap (8) or preventing the arrival of mixture to the burner (4) and the wick (3) by displacing one or both of these components.

The role of the flame in the start-up is to provide the energy required to activate the catalytic process. A valid alternative to the flame for initiating the catalytic combustion is applying heat to the burner (4) by an electric heater, which would allow start-up without removing the external protector (9).

As regards the elements or components, these may vary as follows:
- The active substance can be a volatile organic compound active as an air freshener, insecticide, deodorant, disinfectant, bactericide, therapy, etc., while the fuel can be a short-chain linear alcohol such as isopropanol or ethanol.
- On its part, the burner can be made of porous ceramic with a high resistance to thermal impact, use of materials such as aluminium silicate of the mullite, cordierite and caolinite types, or a mineral of the zeolite group being possible
- As regards the catalyst, it can be consist of a noble metal such as platinum, palladium, rhodium or a binary or tertiary alloy or mixture of these metals, providing the catalytic coating on the burner by impregnating the salt of a noble metal in a liquid solution, followed by a reduction by heat.

## Claims

1. Apparatus for diffusion of active substances with catalytic combustion, based on the simple incorporation of a container (1) that holds a mixture (2) of active substance and fuel in which a wick (3) is placed that leads the mixture to a ceramic piece or burner (4) provided with a catalyst on its surface, **characterised in that** the container (1) incorporates two openings, a first opening (11) meant for refilling the mixture (2) in the container and a second opening (12) meant to allow the wick (3) to pass and to attach the burner (4), the burner (4) designed so that it has a conical-shaped lower end to allow coupling by insertion in the upper end of the wick (3) and so that it is coupled on a metallic support (5) that covers the second opening (12) in which is coupled a centring element (6) for the wick (3), which ensures the vertical position of said wick (3) in the correct position for interfering with the burner (4), being provided at the inlet opening (11) with a refilling cap (7) that closes the container after it is refilled, the container (1) being covered by an external protection (9) that prevents contact with the burner (4) during its use, and provided with orifices that allow circulation of air for combustion and evaporation of the active substances.

2. - Apparatus for diffusion of active substances with catalytic combustion according to claim 1, **characterised in that** when the apparatus is being used a metallic extinguishing cap (8) is placed above the refilling cap (7) to ensure its availability for a later use by placing it on the metallic support (5) and covering the burner (4) to turn off the apparatus.

3. - Apparatus for diffusion of active substances with catalytic combustion according to claim 1, **characterised in that** the metallic support (5) has a substantially greater surface than the surface of the burner (4) that allows dissipating the heat generated in the burner (4) to prevent the container (1) from heating up excessively, allowing the latter to be made of a polymeric material.

4. - Apparatus for diffusion of active substances with catalytic combustion according to claim 1, **characterised in that** the burner (4) can be displaced with respect to the wick (3) to regulate the diffusion of active substance.

5. - Apparatus for diffusion of active substances with catalytic combustion according to claim 1, **characterised in that** it incorporates a thermochromic element (10) on the surface of the metallic support (5) of the ceramic piece that changes colour to indicate an increase in the temperature of the metallic support (5) when the apparatus is in use.

## Patentansprüche

1. Vorrichtung zur Diffusion von Aktivsubstanzen mit katalytischer Verbrennung, basierend auf der einfachen Inkorporation eines Behälters (1), der eine Mischung (2) von Aktivsubstanz und Brennstoff enthält, in welcher ein Docht (3) angeordnet ist, der die Mischung zu einem Keramikteil oder Brenner (4) führt, welcher auf seiner Oberfläche mit einem Katalysator versehen ist, **dadurch gekennzeichnet, dass** der Behälter (1) zwei Öffnungen umfasst, wobei eine erste Öffnung (11) zum Nachfüllen der Mischung (2) in den Behälter gedacht ist und eine zweite Öffnung (12) zum Passierenlassen des Dochtes (3) und Anbringen des Brenners (4) gedacht ist, wobei der Brenner (4) so ausgebildet ist, dass er ein konisch geformtes unteres Ende aufweist, um Kopplung durch Einführen in das obere Ende des Dochtes (3) zu erlauben, und dass er an einen metallischen Halter (5) gekoppelt ist, der die zweite Öffnung (12) bedeckt, in der ein Zentrierelement (6) für den Docht (3) gekoppelt ist, welches die vertikale Position des Dochtes (3) in der korrekten Position für ein Wechselwirken mit dem Brenner (4) gewährleistet, wobei an der Einlassöffnung (11) eine Nachfüllkappe (7) bereitgestellt ist, welche den Behälter nach dem Nachfüllen verschließt, wobei der Behälter (1) durch einen Außenschutz (9) abgedeckt ist, der Kontakt mit dem Brenner (4) während seines Gebrauchs verhindert, und mit Öffnungen versehen ist, welche die Zirkulation von Luft für die Verbrennung und Verdampfung der Aktivsubstanzen erlauben.

2. Vorrichtung zur Diffusion von Aktivsubstanzen mit katalytischer Verbrennung nach Anspruch 1, **dadurch gekennzeichnet, dass** beim Gebrauch der Vorrichtung eine metallische Löschkappe (8) über die Nachfüllkappe (7) platziert wird, um ihre Verfügbarkeit für einen späteren Gebrauch, in dem sie auf den metallischen Halter (5) platziert wird und den Brenner (4) abdeckt, um die Vorrichtung abzustellen, zu gewährleisten.

3. Vorrichtung zur Diffusion von Aktivsubstanzen mit katalytischer Verbrennung nach Anspruch 1, **dadurch gekennzeichnet, dass** der metallische Halter (5) eine wesentlich größere Oberfläche als die Oberfläche des Brenners (4) aufweist, welche Dissipation der in dem Brenner (4) erzeugten Wärme erlaubt, um übermäßiges Aufheizen des Behälters (1) zu verhindern, so dass Letzterer aus einem polymeren Material hergestellt werden kann.

4. Vorrichtung zur Diffusion von Aktivsubstanzen mit katalytischer Verbrennung nach Anspruch 1, **dadurch gekennzeichnet, dass** der Brenner (4) bezüglich des Dochtes (3) versetzt werden kann, um die Aktivsubstanzdiffusion zu regulieren.

5. Vorrichtung zur Diffusion von Aktivsubstanzen mit katalytischer Verbrennung nach Anspruch 1, **dadurch gekennzeichnet, dass** sie ein thermochromes Element (10) an der Oberfläche des metallischen Halters (5) des Keramikteils umfasst, welches die Farbe wechselt, um einen Anstieg der Temperatur des metallischen Halters (5) anzuzeigen, wenn die Vorrichtung in Gebrauch ist.

## Revendications

1. Appareil de diffusion de substances actives à combustion catalytique, basée sur la simple incorporation d'un contenant (1) qui contient un mélange (2) d'une substance active et d'un combustible dans lequel une mèche (3) est placée qui mène le mélange à une pièce céramique ou brûleur (4) prévu avec un catalyseur sur sa surface, **caractérisé en ce que** le contenant (1) incorpore deux ouvertures, une première ouverture (11) créée pour le rechargement du mélange (2) dans le contenant et une seconde ouverture (12) créée pour permettre à la mèche (3) de passer et de s'attacher au brûleur (4), le brûleur (4) étant conçu de sorte à ce qu'il possède une extrémité inférieure de forme conique pour permettre le couplage par insertion dans l'extrémité supérieure de la mèche (3) et de sorte à ce qu'il soit couplé sur un support métallique (5) qui couvre la seconde ouverture (12) dans laquelle est couplée un élément de centrage (6) pour la mèche (3), qui assure la position verticale de ladite mèche (3) dans la position correcte pour interférer avec le brûleur (4), étant prévu à l'ouverture d'entrée (11) avec une capsule de rechargement (7) qui ferme le contenant après qu'il soit rechargé, le contenant (1) étant recouvert par une protection externe (9) qui empêche un contact avec le brûleur (4) pendant son utilisation, et pourvu d'orifices qui permettent une circulation d'air pour une combustion et une évaporation des substances actives.

2. Appareil de diffusion de substances actives avec une combustion catalytique selon la revendication 1, **caractérisé en ce que**, lorsque l'appareil est utilisé, une capsule d'extinction métallique (8) est placée au dessus de la capsule de rechargement (7) pour assurer sa disponibilité pour une utilisation ultérieure en la plaçant sur le support métallique (5) et en couvrant le brûleur (4) pour éteindre l'appareil.

3. Appareil de diffusion de substances actives à combustion catalytique selon la revendication 1, **caractérisé en ce que** le support métallique (5) a une surface substantiellement plus grande que la surface du brûleur (4) qui permet une dissipation de la chaleur générée dans le brûleur (4) pour empêcher au contenant (1) de chauffer excessivement, permettant à ce dernier d'être constitué d'un matériau polymère.

4. Appareil de diffusion de substances actives à combustion catalytique selon la revendication 1, **caractérisé en ce que** le brûleur (4) peut être déplacé par rapport à la mèche (3) pour réguler la diffusion de substance active.

5. Appareil de diffusion de substances actives à combustion catalytique selon la revendication 1, **caractérisé en ce qu'**il incorpore un élément thermochromique (10) sur la surface du support métallique (5) de la pièce céramique qui change de couleur pour indiquer une augmentation dans la température du support métallique (5) lorsque l'appareil est en utilisation.
